# EUROPEAN PATENT APPLICATION

(11) **EP 3 590 361 A1**
(43) Date of publication of application: **08.01.2020**
(21) Application number: 18864110.4
(22) Date of filing: 05.10.2018
(51) Int. Cl.: A23L 33/105, A61K 8/49, A61K 8/60, A61K 8/9789, A61K 9/14, A61K 31/352, A61K 36/28, A61K 47/36, A61P 29/00, A61P 35/00, A61P 37/08, A61P 39/06

(54) **LUTEOLIN-CONTAINING COMPOSITION AND METHOD FOR MANUFACTURING SAME**

(30) Priority: 05.10.2017 JP 2017194890
(71) Applicant: Petroeuroasia Co., Ltd., Shizuoka 411-0903 (JP)
(72) Inventor: TAKAHASHI Hidehiro, Shizuoka 411-0907 (JP)
(74) Representative: Regimbeau
(86) International application number: PCT/JP2018/037376
(87) International publication number: WO 2019/070056

(57) **Abstract**

An objective of the present invention is to provide a water-dispersible, luteolin-containing composition and a method of producing the same. According to the present invention, a water-dispersible powder composition comprising (A) luteolin and (B) a saponin is provided. The luteolin in the form of the plant extract can be mixed. According to the present invention, a method of producing the foregoing luteolin-containing composition, which comprises the steps of performing homogenization process of (A) luteolin and (B) a saponin in water to obtain an emulsion composition; and drying the obtained composition, is also provided.

## Description

### CROSS-REFERENCE TO RELATED APPLICATIONS

This application enjoys the benefits of priority from preceding Japanese Patent Application No. 2017-194890 (filing date: October 5, 2017), the disclosure of which is incorporated herein by reference in its entirety.

### TECHNICAL FIELD

The present invention relates to a luteolin-containing composition and a method of producing the same, more particularly, to a water-dispersible powder composition containing luteolin and a method of producing the same.

### BACKGROUND ART

Luteolin is a type of flavonoids contained in chrysanthemum flowers, peanut shells, perilla fruits, *Perilla frutescens* and the like, and is known to exert various physiologically active effects *in vivo,* such as antioxidant action, anti-allergic action, anti-inflammatory action, anti-cancer action. This is the reason why recent interest has focused on luteolin as a functional material in a wide range of fields of foods, cosmetics and pharmaceuticals.

Luteolin is, however, a substance with poor water solubility. For practical use of luteolin as foods or the like, considerably less solubility of luteolin to water has been the problem. Although a technique in which luteolin was conjugated with cyclodextrin (Patent Document 1) has been suggested so far, there was a limit to how much amount of luteolin can be contained in a composition. In addition to the foregoing technique, a technique of production of a solid composition containing polyphenols, such as luteolin, by using hydrophilic polymer and a nonionic detergent, has been reported (Patent Document 2). However, this technique requires the process of applying heat to melt polyphenols and the nonionic detergent and the like and kneading them, which is accompanied with danger caused by extremely high heating temperature of 350°C, and also has the potential for thermolysis of mixing ingredients. Therefore, the practical use of the technique is anticipated to be challenging.

### Reference List

### Patent Documents

[Patent Document 1] JP 2008-509173 A
[Patent Document 2] WO2017/061627 A1

### SUMMARY OF INVENTION

An objective of the present invention is to provide a water-dispersible, luteolin-containing composition and a method of producing the same.

The present inventor has found currently that a powder composition obtained by performing homogenization process of luteolin, which is a poor-water-solubility substance, with a saponin to obtain an emulsion composition, followed by drying of the obtained emulsion composition, is water-dispersible and possesses an excellent stability after dissolution. The present inventor has further found that, even after preserved, the obtained powder composition excels in water solubility and in stability after dissolution. This result is surprising because despite luteolin's poor water solubility, the obtained luteolin-containing composition can be completely dissolved into water, and moreover the solution is stable. The present invention is based on these findings.

According to the present invention, the following inventions are provided:
[1] A water-dispersible powder composition, comprising: (A) luteolin and (B) a saponin.
[2] The composition according to [1], which comprises the luteolin in the form of plant extract.
[3] The composition according to [2], wherein the plant extract is a chrysanthemum flower extract.
[4] The composition according any one of [1] to [3], wherein the luteolin content is 5% or more by mass.
[5] The composition according to any one of [1] or [4], wherein the (B) saponin is Quillaja saponin.
[6] The composition according to any one of [1] to [5], further comprising: (C) an excipient and/or (D) a thickener.
[7] A food comprising the composition according to any one of [1] to [6].
[8] The food according to [7], wherein the food is in beverage form.
[9] A method of producing the composition according to any one of [1] to [6], comprising the steps of:
   performing homogenization process of (A) luteolin and (B) a saponin in water to obtain an emulsion composition; and
   drying the obtained composition.

According to the present invention, luteolin, which is poor-water-solubility substance, can easily be dispersed in water by mixing it with the saponin, leading to an excellent stability after dissolution as well. The present invention is therefore advantageous in that it can facilitate use of luteolin as foods (especially, beverages) and the like. Further, since saponins are approved for food use and are not allergen, it is also advantageous in respect that the composition of the present invention can be taken without anxiety.

### DETAILED DESCRIPTION OF THE INVENTION

A composition of the present invention comprises (A) luteolin and (b) a saponin.

### (A) Luteolin

Luteolin is a type of flavonoids, and is known to exert various effects, such as antioxidant action, anti-allergic action, anti-inflammatory action, anti-anxiety action, anti-cancer action, improvement action of learning/memory, sleep improvement action, anti-gout action, anti-wrinkle action, improvement action of atopic dermatitis, normalization action of immune function, protective action of hepatopathy, antidepressant action. Luteolin is represented by the following structural formula:

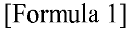

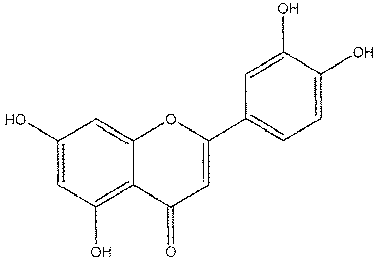

As the luteolin used in the present invention, luteolin extracted from plants, which luteolin may be purified as necessary, may be used. Thus, the luteolin used in the present invention may be in the form of purified one or may be in the form of plant extract (roughly purified one). In the present invention, the luteolin produced by synthesis or luteolin commercially available may also be used. In cases where the luteolin, used in the present invention, in the form of the plant extract is mixed, the compound of the present invention can be restated as a water-dispersible powder composition which comprises: (A') an extract of a luteolin-containing plant and (B) a saponin.

In cases where luteolin is extracted from plants, source plants are not particularly limited as long as they contain luteolin, and for example, chrysanthemum, peanut, perilla, *Perilla frutescens*, garland chrysanthemum, mint, rosemary, celery, parsley, green pepper, apple, chamomile can be used.

Plant extracts containing luteolin include chrysanthemum flower extracts, perilla fruit extracts, peanut shell extracts, *Perilla frutescens-*fruit extracts, celery extracts, parsley extracts, green pepper extracts. Chrysanthemum flowers are rich in luteolin and flavonoids other than luteolin, and do not contain typical of allergens. Thus, in the present invention, preferably, luteolin in the form of chrysanthemum flower extracts can be mixed. Examples of flavonoids other than luteolin include apigenin having the following structural formula:

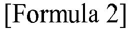

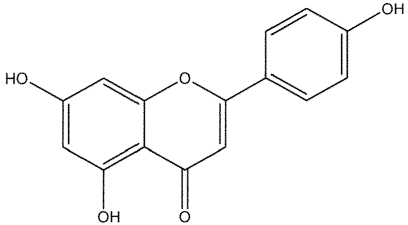

Extraction of luteolin from plants can be performed through a solvent extraction or a supercritical fluid extraction. In the case of the solvent extraction, solvents to be used are not particularly limited, but it is preferred that polar solvents be used. Examples of the polar solvents that can be used include water, methanol, ethanol, isopropanol, acetone, 1,3-butylene glycol, ethylene glycol, propylene glycol, glycerin, acetic acid, ethyl acetate, ether, hexane, and the polar solvent is preferably water, ethanol, more preferably aqueous ethanol. Among these solvents, only one solvent may be used, or a combination of two or more solvents may be used. The obtained extract may be subjected to additional processes, such as concentration, drying, purification. The solvent extraction from luteolin-containing plants is known, e.g., can be performed according to the description of JP2015-212236A.

The luteolin content (in terms of solid content) in the composition of the present invention can be 3% or more by mass, preferably 5% or more by mass or 10% or more by mass. The upper limit of the luteolin content (in terms of solid content) in the composition of the present invention can be, e.g., 18% by mass, 20% by mass or 25% by mass. The luteolin content (in terms of solid content) in the composition of the present invention can be, e.g., 5 to 25% by mass or 5 to 20% by mass. Since the luteolin content of the composition of the present invention can be set to 5% or more by mass while maintaining dispersibility of luteolin to water, the composition of the present invention is advantageous in that it can supply more amount of luteolin to foods and the like even if supplying the same additive amount of luteolin.

### (B) Saponins

Saponins are glycosides in which a glucose binds to a steroid or triterpene, which are present in plants, and have foaming property. Classifying saponins based on their sources, for example, saponins include Quillaja saponin, Yucca saponin, soy saponin, carrot saponin and the like. In the present invention, saponins that are roughly purified or purified from a plant body may be used. In the present invention, for example, a quillaja extract and a yucca form extract can be used as Quillaja saponin and Yucca saponin, respectively.

In the present invention, any one of the saponins may be used alone or in combination with another saponin, and it is preferred that Quillaja saponin be used alone, or alternatively Quillaja saponin be used in combination with another saponin.

The saponin content (in terms of solid content) in the composition of the present invention can be an amount that enables luteolin to be dispersible to water, e.g., can be 0.5 to 30% by mass, preferably 1 to 10% by mass.

Regarding the mixing ratio (by mass based on solid content) of saponin to luteolin, the lower limit can be 0.3 (preferably 0.4, more preferably 0.6, still more preferably 1) in light of enhancement of the solubility to water of luteolin. Regarding the mixing ratio (by mass based on solid content) of saponin to luteolin, the upper limit of three or four in consideration of influence of bitter taste and the like of saponins. The mixing ratio (by mass based on solid content) of saponin to luteolin can be, for example, 0.3 to 4 or 0.4 to 3. Note that in cases where the luteolin in the form of the plant extract is mixed, the mixing ratio can be determined based on the luteolin content in the extract.

A method for measuring the luteolin and saponin contents is not particularly limited, and they can be measured by using, e.g., high-performance liquid chromatography (HPLC), gas chromatography.

The composition of the present invention may further comprise one or two or more components selected from the group consisting of (C) an excipient, (D) a thickener and (E) an antioxidant.

### (C) Excipient

In the present invention, one or two or more excipients can be used to provide the composition of the present invention in powder form. As excipients that can be used for the present invention, any excipients that are acceptable for foods and the like may be used, and examples of excipients include starches, processed starches, saccharides, celluloses. Among them, examples of starches include corn starch, wheat starch, potato starch, and examples of processed starches include dextrin, maltodextrin, powdered corn syrup, solubilized starch. Examples of saccharides include sugar alcohols (e.g., xylitol, maltitol, inositol) in addition to monosaccharides and disaccharides (e.g., lactose, xylose). In the present invention, dextrin and/or maltodextrin can be preferably used as the excipient.

The excipient content (in terms of solid content) in the composition of the present invention can be 5 to 60% by mass, preferably 10 to 50% by mass.

### (D) Thickener

In the present invention, one or two or more thickeners can be used to provide the composition of the present invention in powder form. As thickeners that can be used for the present invention, any thickeners that are acceptable for foods and the like may be used, and examples of thickeners include gum arabic, arabinogalactan, alginic acid, welan gum, elemi resin, artemisia sphaerocephala seed gum, curdlan, cassia gum, sodium caseinate, carrageenan, karaya gum, carob bean gum, xanthan gum, chitin, chitosan, guar gum, glucosamine, psyllium seed gum, gellan gum, tamarind gum, *Hibiscus manihot,* tragacanth gum, *Bacillus natto* gum, furcellaran, pullulan, pectin, Macrophomopsis gum, methylcellulose, *Colpomenia sinuosa* extract. In the present invention, gum arabic can be preferably used as the thickener.

The thickener content (in terms of solid content) in the composition of the present invention can be 0.1 to 20% by mass, preferably 2 to 15% by mass.

### (E) Antioxidant

In the present invention, one or two or more antioxidants can be used to suppress oxidation and decomposition of luteolin contained in the composition of the present invention. As antioxidants that can be used for the present invention, any antioxidants that are acceptable for foods and the like may be used, and examples of antioxidants include ascorbic acid and ester and salt thereof, sodium sulfite, calcium disodium ethylenediaminetetracetate, disodium ethylenediaminetetracetate, L-cysteine hydrochloride, dibutylhydroxytoluene, d-α-tocopherol acetate, d-tocopherols, tocotrienol, mixed tocopherols, catechin, quercetin, rutin, ferulic acid, gallic acid, rice bran oil extract, sage extract, raw coffee bean extract, sunflower seed extract, grape seed extract, sesame oil unsaponifiables, tea extract, propolis extract, bayberry extract, hego-ginkgo leaf extract, enju extract. Among them, examples of the ascorbic acid and ester and salt thereof include L-ascorbic acid, L-ascorbic acid ester, sodium L-ascorbate, L-ascorbyl palmitate. In the present invention, the ascorbic acids and/or salt thereof can be preferably used as the antioxidant.

The antioxidant content (in terms of solid content) in the composition of the present invention can be 0 to 30% by mass, preferably 2 to 25% by mass.

To the composition of the present invention, any components that are acceptable for foods and the like other than the foregoing (A), (B), (C), (D) and (E) may be added, and such components include flavors, preservatives.

The composition of the present invention can be produced by homogenization of raw materials as described later. Accordingly, the composition of the present invention can include the components (A) and (B) above and optionally, the components (C), (D) and (E) above and any other components, in a homogenized state.

The composition of the present invention is also an emulsified composition obtained by drying an emulsified composition of luteolin, which is water-dispersible. Herein, whether or not a composition is "water-dispersible" can be evaluated by visual observation of a solution state obtained by adding 0.2 g of the composition (in terms of solid content) into 100 mL of water at room temperature and then stirring and mixing the mixture. Specifically, when the total amount of the composition having been added is dissolved in water and no suspensions nor precipitates are generated, the composition can be determined as "water-dispersible."

Since the composition of the present invention is a water-dispersible, luteolin-containing composition, it allows for adding luteolin or an extract of luteolin-containing plants, as a raw material, to a food to produce the food in which the luteolin is mixed homogeneously, in hopes of various functions of luteolin or an extract of luteolin-containing plants, such as a chrysanthemum flower extract. The composition of the present invention also allows for adding luteolin or an extract of luteolin-containing plants, as a raw material, not only to a food but also to a feed or pet food to produce the feed and pet food in which the luteolin is mixed homogeneously. That is, the present invention can provide a food, feed and pet food in which the composition of the present invention is mixed.

The food into which the composition of the present invention can be mixed is not particularly limited and includes waters (e.g., drinking water, hydrogen water, mineral water, near water), soft, refreshing liquids (e.g., fruit juice, vegetable juice, drinks including fruit and vegetable juice, carbonated drinks, functional drinks, sports drinks, non-alcoholic drinks), tea drinks (e.g., green tea, black tea, Chinese teas, roasted-barley tea, buckwheat tea, mate, blended tea), coffee drinks (e.g., canned coffee, instant coffee), cocoa drinks, lactic acid bacteria drinks, Japanese Aojiru, soymilk drinks, dairy products (e.g., milk, milk beverage, processed milk, yogurt), energy drinks, jelly drinks, soups, alcoholic drinks (e.g., beer, low-malt beer, whiskey, bourbon, spirits, liqueur, wine, fruit wine, Sake, Shochu, other miscellaneous liquors), carbohydrate-containing foods (e.g., rice, noodles, breads, pasta), western-style confectionery (e.g., biscuit, cracker, cookie, waffle, pie), Japanese-style confectionery (e.g., rice cracker, sweetened bean paste bun, adzukibean jelly), chewing gums, candies, frozen desserts (e.g., ice cream, and sherbet).

The food into which the composition of the present invention can be mixed also may be in liquid form, such as drink, fluid diet, may be in paste, semiliquid, or gel form, or may be in solid or powder form. Since the composition of the present invention excels not only in solubility to water but also in stability after dissolved into water, it is advantageous in that the food into which the composition of the present invention is mixed can be provided in beverage form despite of containing luteolin, which is poor-water-solubility.

A mixing amount of the composition of the present invention in the food of the present invention can be determined using a daily intake of luteolin as a rough target. A daily effective intake of luteolin for human in cases where its antioxidant activity is desired is 1 to 100 mg (preferably, 5 to 50 mg), and the composition of the present invention can be mixed into the food of the present invention such that such a daily effective intake of luteolin for human can be taken. In this case, the food of the present invention may be packaged in such a way that the daily effective intake of luteolin can be taken. The food may be in package form of a single serve or of being divided into multiple packages as long as the daily effective intake can be taken. In cases where luteolin is provided in the package form, it is desirable that statements for the intake be printed on the package such that the daily effective intake can be taken, or the package be provided together with a package insert including such statements. Further, in cases where the daily effective intake is provided in the multiple packages, for convenience in intake, a set of the multiple packages including the daily effective intake also can be provided.

Since the composition of the present invention is a water-dispersible, luteolin-containing composition, it allows for adding luteolin or an extract of luteolin-containing plants, as a raw material, to a cosmetic or external skin preparation to produce the cosmetic or pharmaceutical in which the luteolin is mixed homogeneously in hopes of various functions of luteolin or an extract of luteolin-containing plants, such as a chrysanthemum flower extract. That is, the present invention can provide a cosmetic (e.g., skin care cosmetics) and pharmaceutical (e.g., external skin preparation), to both of which the composition of the present invention is mixed.

The composition of the present invention can be produced by performing homogenization process of raw materials and dry process of the obtained emulsion composition.

The homogenization process of raw materials can be performed by first adding the raw materials including luteolin and a saponin into water and mixing the mixture and optionally further adding an excipient, a thickener, an antioxidant and/or any other components to the mixture and mixing the mixture, followed by homogenization process of the obtained mixture.

The homogenization process is a process for reducing size of component particles included in the mixture and homogenizing the particles. The homogenization process can be performed with a homogenizer (e.g., Milder (manufactured by Pacific Machinery & Engineering Co., Ltd.), Silverson homogenizer (manufactured by SILVERSON NIPPON CO., LTD.)), high-pressure homogenizer (e.g., Homogenizer (manufactured by SANWA MACHINE CO., INC.), High-pressure homogenizer (manufactured by SANMARU MACHINERY CO., LTD.,)) or the like. In the case of using homogenizers, the homogenization can be performed under the condition of 25 to 40 m/s. In the case of using high-pressure homogenizers, the homogenization can be performed under the condition of 50 to 150 MPa, and may be performed with further strong force being applied. By the homogenization process, the luteolin-containing emulsion composition can be obtained. In the emulsion composition, the luteolin is in a homogenized state, i.e., in a state where it is dispersed in water.

The dry process of the emulsion composition can be performed by a known method, such as spray drying, freeze drying. By the dry process, a powder luteolin-containing composition can be obtained, and the obtained powder luteolin-containing composition includes luteolin with being homogenized with other components.

### EXAMPLES

The present invention will now be more specifically described based on the following examples, but the present invention is not to be limited to these examples.

### Example 1: Production of luteolin-containing powder composition in which Quillaja saponin is mixed (1)

According to the mixing amount in Table 1, the luteolin-containing powder compositions in which Quillaja saponin was mixed were produced. Specifically, Quillaja saponin was mixed into 300 mL of pure water and heated to 80°C, and then to the resultant solution, luteolin, dextrin and gum arabic were added, mixed and dissolved. The mixture was emulsified using a high-pressure homogenizer (manufactured by SANMARU MACHINERY CO., LTD., 500 kg/cm², repeated three times). After the emulsification, using a spray dryer (Mini Spray Dryer GB22, manufactured by Yamato Scientific Co., Ltd.), luteolin dry powder in which Quillaja saponin was mixed was prepared.

### [Table 1]

**Table 1: Luteolin-containing powder composition in which Quillaja saponin is mixed**

| Experimental section | Mixed components | |
|---|---|---|
| 1 | Luteolin: | 100 g |
| | Quillaja saponin: | 10 g (100 g of Quillajanin S-100 manufactured by MARUZEN PHARMACEUTICALS CO., LTD.) |
| | Dextrin: | 100 g |
| | Gum arabic: | 30 g |
| 2 | Luteolin: | 100 g |
| | Quillaja saponin: | 15 g (150 g of Quillajanin S-100 manufactured by MARUZEN PHARMACEUTICALS CO., LTD.) |
| | Dextrin: | 100 g |
| | Gum arabic: | 30 g |
| 3 | Luteolin: | 100 g |
| | Quillaja saponin: | 20 g (200 g of Quillajanin S-100 manufactured by MARUZEN PHARMACEUTICALS CO., LTD.) |
| | Dextrin: | 100 g |
| | Gum arabic: | 30 g |
| 4 | Luteolin: | 100 g |
| | Quillaja saponin: | 30 g (300 g of Quillajanin S-100 manufactured by MARUZEN PHARMACEUTICALS CO., LTD.) |
| | Dextrin: | 100 g |
| | Gum arabic: | 30 g |

| | | |
|---|---|---|
| Luteolin: Chrysanthemum flower extract (manufactured by HANGZHOU SKYHERB TECHNOLOGIES Co., Ltd. (China), luteolin content: 50%) Dextrin: manufactured by San-ei Sucrochemical Co., Ltd. Gum arabic: manufactured by NIPPON FUNMATSU YAKUHIN Co., LTD. | | |

For the obtained luteolin dry powder, state of the powder, solubility to water, and stability after dissolved and then settled for eight hours were evaluated. The state of the powder and the solubility to water were classified into three levels (A: good, B: normal, C: bad) and evaluated through visual observation. The solubility to water was also observed after addition of 0.2 g of the luteolin dry powder into 100 mL of water at room temperature and stirring and mixing the mixture.

The results are as shown in Table 2.

### [Table 2]

**Table 2: Evaluation for luteolin-containing powder composition in which Quillaja saponin is mixed**

| Experimental section | State of powder | Solubility to water | Stability after settled for 8 hrs |
|---|---|---|---|
| 1 | A | C | Suspensions were generated. |
| 2 | A | A | Suspensions were slightly generated. |
| 3 | A | A | The state where the composition had been dissolved was maintained. |
| 4 | A | A | The state where the composition had been dissolved was maintained. |

Furthermore, the obtained luteolin dry powder was transferred by 10 g to individual glass bottles, each of which was stored at 55°C. On the 14th day, state of the powder, solubility to water, stability after dissolved and then settled for eight hours, and the remaining amount of luteolin were evaluated. The solubility to water was classified into three levels (A: good, B: normal, C: bad) and evaluated through visual observation. The solubility to water was also observed after addition of 0.2 g of the luteolin dry powder into 100 mL of water at room temperature and stirring and mixing the mixture.

The results are as shown in Table 3.

### [Table 3]

**Table 3: Preservation stability of luteolin-containing powder composition in which Quillaja saponin is mixed**

| Experimental section | State of powder | Solubility to water | Stability after settled for 8 hrs | Remaining amount of luteolin |
|---|---|---|---|---|
| 1 | No changes | C | Precipitates were genera ted. | 90% |
| 2 | No changes | A | The state where the composition had been dissolved was maintained. | 92% |
| 3 | No changes | A | The state where the composition had been dissolved was maintained. | 93% |
| 4 | No changes | A | The state where the composition had been dissolved was maintained. | 93% |

As described above, the luteolin-containing powder compositions in which Quillaja saponin is mixed have excelled not only in solubility to water but also in stability after the dissolution.

### Example 2: Production of luteolin-containing powder composition in which Quillaja saponin is mixed (2)

According to the mixing amount in Table 4, the luteolin-containing powder compositions in which Quillaja saponin was mixed were produced. Specifically, Quillaja saponin was mixed into 300 mL of pure water and heated to 80°C, and then to the resultant solution, luteolin, dextrin and gum arabic were added, mixed and dissolved. The mixture was emulsified using a high-pressure homogenizer (manufactured by SANMARU MACHINERY CO., LTD., 500 kg/cm², repeated three times). After the emulsification, using a spray dryer (Mini Spray Dryer GB22, manufactured by Yamato Scientific Co., Ltd.), luteolin dry powder in which Quillaja saponin was mixed was prepared.

### [Table 4]

**Table 4: Luteolin-containing powder composition in which Quillaja saponin is mixed**

| Experimental section | Mixed components | |
|---|---|---|
| 5 | Luteolin: | 50 g |
| | Quillaja saponin: | 10 g (100g of Quillajanin S-100 manufactured by MARUZEN PHARMACEUTICALS CO., LTD.) |
| | Dextrin: | 100 g |
| | Gum arabic: | 30 g |
| 6 | Luteolin: | 50 g |
| | Quillaja saponin: | 15 g (150g of Quillajanin S-100 manufactured by MARUZEN PHARMACEUTICALS CO., LTD.) |
| | Dextrin: | 100 g |
| | Gum arabic: | 30 g |
| 7 | Luteolin: | 50 g |
| | Quillaja saponin: | 20 g (200 g of Quillajanin S-100 manufactured by MARUZEN PHARMACEUTICALS CO., LTD.) |
| | Dextrin: | 100 g |
| | Gum arabic: | 30 g |
| 8 | Luteolin: | 50 g |
| | Quillaja saponin: | 30 g (300 g of Quillajanin S-100 manufactured by MARUZEN PHARMACEUTICALS CO., LTD.) |
| | Dextrin: | 100 g |
| | Gum arabic: | 30 g |

| | | |
|---|---|---|
| Luteolin: KS-1906, Luteolin 98% powder from Peanut Shell (manufactured by SHAANXI KINGSCI BIOTECHNOLOGY Co., Ltd. (China), origin: peanut shell, luteolin content: 98%) Dextrin: manufactured by San-ei Sucrochemical Co., Ltd. Gum arabic: manufactured by NIPPON FUNMATSU YAKUHIN Co., LTD. | | |

For the obtained luteolin dry powder, state of the powder, solubility to water, and stability after dissolved and then settled for eight hours were evaluated. The state of the powder and the solubility to water were classified into three levels (A: good, B: normal, C: bad) and evaluated through visual observation. The solubility to water was also observed after addition of 0.2 g of the luteolin dry powder into 100 mL of water at room temperature and stirring and mixing the mixture.

The results are as shown in Table 5.

### [Table 5]

**Table 5: Evaluation for luteolin-containing powder composition in which Quillaja saponin is mixed**

| Experimental section | State of powder | Solubility to water | Stability after settled for 8 hrs |
|---|---|---|---|
| 5 | A | C | Suspensions were generated. |
| 6 | A | A | The state where the composition had been dissolved was maintained. |
| 7 | A | A | The state where the composition had been dissolved was maintained. |
| 8 | A | A | The state where the composition had been dissolved was maintained. |

Furthermore, the obtained luteolin dry powder was transferred by 10 g to individual glass bottles, each of which was stored at 55°C. On the 14th day, state of the powder, solubility to water, stability after dissolved and then settled for eight hours, and the remaining amount of luteolin were evaluated. The solubility to water was classified into three levels (A: good, B: normal, C: bad) and evaluated through visual observation. The solubility to water was also observed after addition of 0.2 g of the luteolin dry powder into 100 mL of water at room temperature and stirring and mixing the mixture.

The results are as shown in Table 6.

### [Table 6]

**Table 6: Preservation stability of luteolin-containing powder composition in which Quillaja saponin is mixed**

| Experimental section | State of powder | Solubility to water | Stability after settled for 8 hrs | Remaining amount of luteolin |
|---|---|---|---|---|
| 5 | No changes | C | Precipitates were generated. | 91% |
| 6 | No changes | A | Suspensions were slightly generated. | 92% |
| 7 | No changes | A | The state where the composition had been dissolved was maintained. | 91% |
| 8 | No changes | A | The state where the composition had been dissolved was maintained. | 93% |

As described above, the luteolin-containing powder compositions in which Quillaja saponin is mixed have excelled not only in solubility to water but also in stability after the dissolution.

### Example 3: Production of luteolin-containing powder composition in which Quillaja saponin is mixed (3)

According to the mixing amount in Table 7, the luteolin-containing powder compositions in which Quillaja saponin was mixed were produced. Specifically, Quillaja saponin was mixed into 300 mL of pure water and heated to 80°C, and then to the resultant solution, luteolin, dextrin and gum arabic were added, mixed and dissolved. The mixture was emulsified using a high-pressure homogenizer (manufactured by SANMARU MACHINERY CO., LTD., 500 kg/cm², repeated three times). After the emulsification, using a spray dryer (Mini Spray Dryer GB22, manufactured by Yamato Scientific Co., Ltd.), luteolin dry powder in which Quillaja saponin was mixed was prepared.

### [Table 7]

**Table 7: Luteolin-containing powder composition in which Quillaja saponin is mixed**

| Experimental section | Mixed components | |
|---|---|---|
| 9 | Luteolin: | 100 g |
| | Quillaja saponin: | 3 g (30g of Quillajanin S-100 manufactured by MARUZEN PHARMACEUTICALS CO., LTD.) |
| | Dextrin: | 50 g |
| | Gum arabic: | 30 g |
| 10 | Luteolin: | 100 g |
| | Quillaja saponin: | 5 g (50 g of Quillajanin S-100 manufactured by MARUZEN PHARMACEUTICALS CO., LTD.) |
| | Dextrin: | 50 g |
| | Gum arabic: | 30 g |
| 11 | Luteolin: | 100 g |
| | Quillaja saponin: | 10 g (100 g of Quillajanin S-100 manufactured by MARUZEN PHARMACEUTICALS CO., LTD.) |
| | Dextrin: | 50 g |
| | Gum arabic: | 30 g |
| 12 | Luteolin: | 100 g |
| | Quillaja saponin: | 20 g (200 g of Quillajanin S-100 manufactured by MARUZEN PHARMACEUTICALS CO., LTD.) |
| | Dextrin: | 50 g |
| | Gum arabic: | 30 g |

| | | |
|---|---|---|
| Luteolin: "KIKU FLOWER EXTRACT-P" (manufactured by Oryza Oil&Fat Chemical Co., Ltd.) (containing an aqueous ethanol extract from chrysanthemum flowers, luteolin content: 10%) Dextrin: manufactured by San-ei Sucrochemical Co., Ltd. Gum arabic: manufactured by NIPPON FUNMATSU YAKUHIN Co., LTD. | | |

For the obtained luteolin dry powder, state of the powder, solubility to water, and stability after dissolved and then settled for eight hours were evaluated. The state of the powder and the solubility to water were classified into three levels (A: good, B: normal, C: bad) and evaluated through visual observation. The solubility to water was also observed after addition of 0.2 g of the luteolin dry powder into 100 mL of water at room temperature and stirring and mixing the mixture.

The results are as shown in Table 8.

### [Table 8]

**Table 8: Evaluation for luteolin-containing powder composition in which Quillaja saponin is mixed**

| Experimental section | State of powder | Solubility to water | Stability after settled for 8 hrs |
|---|---|---|---|
| 9 | A | C | Suspensions were generated. |
| 10 | A | A | The state where the composition had been dissolved was maintained. |
| 11 | A | A | The state where the composition had been dissolved was maintained. |
| 12 | A | A | The state where the composition had been dissolved was maintained. |

Furthermore, the obtained luteolin dry powder was transferred by 10 g to individual glass bottles, each of which was stored at 55°C. On the 14th day, state of the powder, solubility to water, stability after dissolved and then settled for eight hours, and the remaining amount of luteolin were evaluated. The solubility to water was classified into three levels (A: good, B: normal, C: bad) and evaluated through visual observation. The solubility to water was also observed after addition of 0.2 g of the luteolin dry powder into 100 mL of water at room temperature and stirring and mixing the mixture.

The results are as shown in Table 9.

### [Table 9]

**Table 9: Preservation stability of luteolin-containing powder composition in which Quillaja saponin is mixed**

| Experimental section | State of powder | Solubility to water | Stability after settled for 8 hrs | Remaining amount of luteolin |
|---|---|---|---|---|
| 9 | No changes | C | Precipitates were generated. | 90% |
| 10 | No changes | A | The state where the composition had been dissolved was maintained. | 92% |
| 11 | No changes | A | The state where the composition had been dissolved was maintained. | 93% |
| 12 | No changes | A | The state where the composition had been dissolved was maintained. | 93% |

As described above, the luteolin-containing powder compositions in which Quillaja saponin is mixed have excelled not only in solubility to water but also in stability after the dissolution.

### Example 4: Production of luteolin-containing powder composition in which Yucca saponin is mixed

According to the mixing amount in Table 10, the luteolin-containing powder compositions in which Yucca saponin was mixed were produced. Specifically, Yucca saponin was mixed into 300 mL of pure water and heated to 80°C, and then to the resultant solution, luteolin, dextrin and gum arabic were added, mixed and dissolved. The mixture was emulsified using a high-pressure homogenizer (manufactured by SANMARU MACHINERY CO., LTD., 500 kg/cm², repeated three times). After the emulsification, using a spray dryer (Mini Spray Dryer GB22, manufactured by Yamato Scientific Co., Ltd.), luteolin dry powder in which Yucca saponin was mixed was prepared.

### [Table 10]

**Table 10: Luteolin-containing powder composition in which Yucca saponin is mixed**

| Experimental section | Mixed components | |
|---|---|---|
| 13 | Luteolin: | 50 g |
| | Yucca saponin: | 10 g (20 g of Yucca Sarasaponin 50M manufactured by Watahan Trading Co., Ltd.) |
| | Dextrin: | 100 g |
| | Gum arabic: | 30 g |
| 14 | Luteolin: | 50 g |
| | Yucca saponin: | 20 g (40 g of Yucca Sarasaponin 50M manufactured by Watahan Trading Co., Ltd.) |
| | Dextrin: | 100 g |
| | Gum arabic: | 30 g |
| 15 | Luteolin: | 50 g |
| | Yucca saponin: | 50 g (100 g of Yucca Sarasaponin 50M manufactured by Watahan Trading Co., Ltd.) |
| | Dextrin: | 100 g |
| | Gum arabic: | 30 g |
| 16 | Luteolin: | 50 g |
| | Yucca saponin: | 75 g (150 g of Yucca Sarasaponin 50M manufactured by Watahan Trading Co., Ltd.) |
| | Dextrin: | 100 g |
| | Gum arabic: | 30 g |
| 17 | Luteolin: | 50 g |
| | Yucca saponin: | 100 g (200 g of Yucca Sarasaponin 50M manufactured by Watahan Trading Co., Ltd.) |
| | Dextrin: | 100 g |
| | Gum arabic: | 30 g |

| | | |
|---|---|---|
| Luteolin: KS-1906, Luteolin 98% powder from Peanut Shell (manufactured by SHAANXI KINGSCI BIOTECHNOLOGY Co., Ltd. (China), origin: peanut shell, luteolin content: 98%) Dextrin: manufactured by San-ei Sucrochemical Co., Ltd. Gum arabic: manufactured by NIPPON FUNMATSU YAKUHIN Co., LTD. | | |

The obtained luteolin dry powder was transferred by 10 g to individual glass bottles, each of which was stored at 55°C. On the 14th day, state of the powder, solubility to water, stability after dissolved and then settled for eight hours, and the remaining amount of luteolin were evaluated. The solubility to water was classified into three levels (A: good, B: normal, C: bad) and evaluated through visual observation. The solubility to water was also observed after addition of 0.2 g of the luteolin dry powder into 100 mL of water at room temperature and stirring and mixing the mixture.

The results are as shown in Table 11.

### [Table 11]

**Table 11: Preservation stability of luteolin-containing powder composition in which Yucca saponin is mixed**

| Experimental section | State of powder | Solubility to water | Stability after settled for 8 hrs | Remaining amount of luteolin |
|---|---|---|---|---|
| 13 | No changes | C | Suspensions and precipitates were generated. | 90% |
| 14 | No changes | B | Precipitates were generated. | 90% |
| 15 | No changes | A | Precipitates were generated. | 91% |
| 16 | No changes | A | The state where the composition had been dissolved was maintained. | 91% |
| 17 | No changes | A | The state where the composition had been dissolved was maintained. | 92% |

As described above, the luteolin-containing powder compositions in which Yucca saponin is mixed have excelled in solubility to water and in stability after the dissolution.

### Examples 5: Production of beverage

For a 50 mL of beverage, using mix described in Table 12, a soft beverage (pH 3.6) in which the composition of the present invention was mixed was produced.

### [Table 12]

**Table 12: Mixed components for beverage**

| Component | Mixing amount |
|---|---|
| Composition of the present invention (Example 1, Experimental section 4) | 250 mg (23.6mg as luteolin) |
| Reduced Maltose Starch Syrup (manufactured by Mitsubishi Shoji Foodtech Co., Ltd.) | 1200 mg |
| Sodium Citrate (manufactured by FUSO CHEMICAL CO., LTD.) | 150 mg |
| Sodium Ascorbate (manufactured by Royal DSM) | 100 mg |

The produced beverage was transferred by 50 mL to individual brown bottles of 50 mL, each of which was heated and sterilized at center temperature of 85°C for 60 minutes. The bottles of beverage were stored at 40°C, and their coloring, taste, and remaining amount of luteolin were confirmed in the first month, and the second month. The results are as shown in Table 13.

### [Table 13]

**Table 13: Preservation stability of beverage**

| | Coloring | Taste | Concentration of luteolin | Remaining rate |
|---|---|---|---|---|
| At the beginning of preservation | Brown emulsion | Sour sweetness | 0.91 mg/mL | 100% |
| 1st Month | No changes | No changes | 0.90 mg/mL | 98.9% |
| 2nd Month | No changes | No changes | 0.88 mg/mL | 96.7% |

Even after preservation at 40°C for two months, the produced beverage did not change in coloring and taste, and also maintained almost the same concentration of luteolin as the concentration at the beginning of the preservation. Since the preservation stability test at 40°C for two months is equivalent to a preservation stability test at room temperature for two years, it has been indicated that the beverage in which the composition of the present invention is mixed has a high preservation stability in terms of quality and concentration of luteolin.

## Claims

1. A water-dispersible powder composition, comprising: (A) luteolin and (B) a saponin.

2. The composition according to claim 1, which comprises the luteolin in the form of plant extract.

3. The composition according to claim 2, wherein the plant extract is a chrysanthemum flower extract.

4. The composition according to any one of claims 1 to 3, wherein the luteolin content is 5% or more by mass.

5. The composition according to any one of claims 1 or 4, wherein the (B) saponin is Quillaja saponin.

6. The composition according to any one of claims 1 to 5, further comprising: (C) an excipient and/or (D) a thickener.

7. A food comprising the composition according to any one of claims 1 to 6.

8. The food according to claim 7, wherein the food is in beverage form.

9. A method of producing the composition according to any one of claims 1 to 6, comprising the steps of:
performing homogenization process of (A) luteolin and (B) a saponin in water to obtain an emulsion composition; and
drying the obtained composition.
